Europäisches Patentamt

(19) European Patent Office

Office européen des brevets

(11) Publication number: **0 050 632**
**B1**

(12) **EUROPEAN PATENT SPECIFICATION**

(45) Date of publication of patent specification: 20.05.87

(51) Int. Cl.⁴: **A 61 B 10/00**

(21) Application number: 81901104.0

(22) Date of filing: 13.04.81

(86) International application number:
PCT/US81/00475

(87) International publication number:
WO 81/02974 29.10.81 Gazette 81/25

(54) **PAP SMEAR T-ZONE SAMPLER.**

(30) Priority: 21.04.80 US 142254
22.05.80 US 152375

(43) Date of publication of application:
05.05.82 Bulletin 82/18

(45) Publication of the grant of the patent:
20.05.87 Bulletin 87/21

(84) Designated Contracting States:
AT CH DE FR GB LI LU NL SE

(56) References cited:
US-A-2 471 088
US-A-3 088 454
US-A-3 438 366
US-A-3 485 236
US-A-3 592 186
US-A-3 640 268
US-A-3 796 211
US-A-4 016 865
US-A-4 043 322
US-A-4 078 656
US-E- 27 915

GYNECOLOGICAL PATHOLOGY, CONF. BY
AMER. COLL. OF BO: & GYN. at SAN
FRANCISCO, CALIFORNIA., June 7-9, 1979,
PP.1-11&28-45

(73) Proprietor: **Accupap Inc.**
**120 Northgate Plaza Nr. 345**
**Seattle Washington 98125 (US)**

(72) Inventor: **Hasselbrack, Robert**
**13201 9th Northwest**
**Seattle, WA 98177 (US)**

(74) Representative: **Everitt, Christopher James**
**Wilders et al**
**F. J. CLEVELAND & COMPANY**
**40/43 Chancery Lane**
**London WC2A 1JQ (GB)**

(56) References cited:
**SURG., GYN. & OB.**
**ACTA CYTOLO.**
**OBSTET. & GYNECOL.**
**KOSS, L.G., DIAG. CYTOL. AND ITS**
**HISTORICAL BASIS, LIPPINCOTT, 1979, (176-81)**

**AM. J. CLIN. PATH., 73 (2); 202-216, February 1980**

**CANCER, 18 (11): 1474-1478, November 1965**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Technical Field

Our invention relates to a cytological sampling instrument for collecting cells exfoliated from the uterine cervix.

### Brief Description of the Background Art Drawings

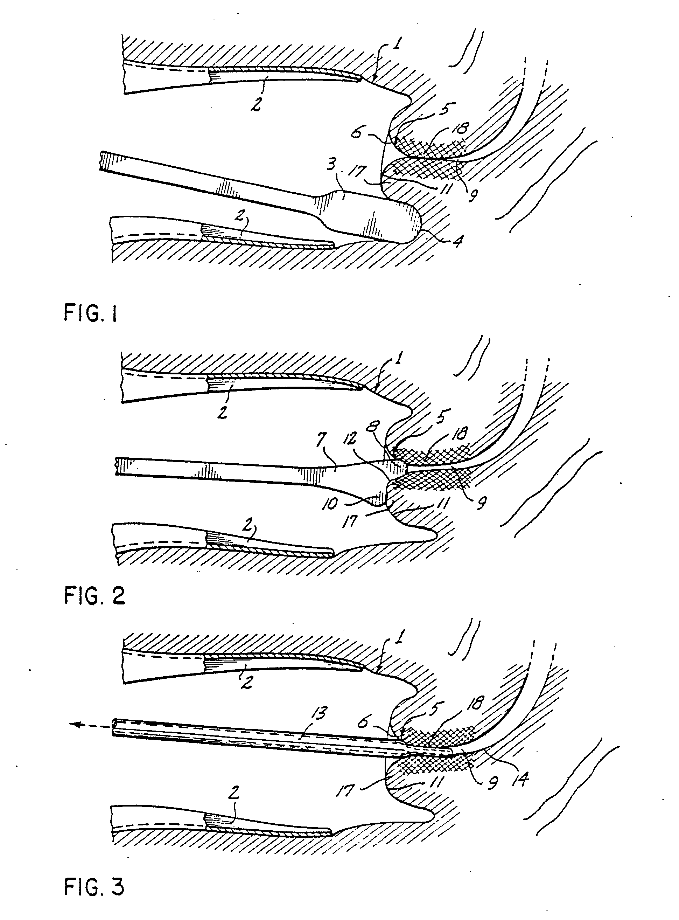

The background art is described below with reference to the accompanying Figures 1, 2, 3 and 4 which are corresponding, somewhat diagrammatic, generally axial sections through a vaginal cavity showing in elevation the most popular prior art cytological sampling instruments currently in use.

### Background Art

In each of the known cervical cytological sampling methods the object is to collect a large number of cells that originated at the uterine cervix, and to a lesser degree, at the uterus, to be deposited on a slide and "fixed" by application of fixative for preservation of the cells. After suitable processing, subsequent microscopic examination in a medical laboratory reveals whether or not abnormal cells are present which are indicative of cancer or lesions accepted as being precursors of cancer.

As illustrated in Figure 1, in one method the vagina 1 is distended by a speculum 2 enabling the portio vaginalis (vaginal portion) 11 of the cervix to be viewed, and a paddle-like scraper 3 is used to collect exfoliated cells from the posterior fornix 4 of the vagina, that is, from the "vaginal pool". This is the area selected for sampling by Papanicolaou because cells exfoliated from virtually all areas of the cervix and from the uterus gather in the vaginal pool, though Papanicolaou proposed sampling this region by aspiration. Unfortunately, however, cells begin deteriorating immediately upon exfoliation to the point where at least a large proportion of the cells obtained in a vaginal pool smear have little or no diagnostic value. In addition, while large numbers of cells usually are obtained, the origins of the cells present in the vaginal pool at any given moment are not known so that there is no assurance that cells from areas prone to cancer will be obtained. It is now recognized that a false negative rate of about 50% can be expected for vaginal pool smears, that is, even after examination about one-half of the cases of invasive cervical cancer and cervical intraepithelial neoplasia ("CIN") remain undetected. Nevertheless, since vaginal pool smears are quickly, easily and inexpensively taken, they still are used to a large extent, particularly when funds for screening a large population are limited.

As early as 1947, the year in which the application resulting in Ayre United States patent No. 2,471,088 was filed, it was recognized that the vast majority of lesions resulting in invasive cervical cancer originate at the undulating circumferential border 5 between the squamous cells of the ectocervical epithelium 17 and the columnar cells of endocervical epithelium 18, which border is referred to as the squamo-columnar junction, the transformation zone of the "T-zone". Though the T-zone is variously located in different women, usually it is at or closely adjacent to the external os 6. It is extremely important that cells from this area be present in a sample.

Ayre invented the specially designed scraper 7 shown in Figure 2 to be used for scraping the entire circumferential extent of the T-zone for early detection of cell abnormalities. In general, the Ayre scraper has two lobes including a frontal lobe 8 insertable slightly into the endocervical canal 9 and an adjacent lateral lobe 10 abuttable against the vaginal portion or ring 11 of the cervix. The frontal lobe 8 acts as a pivot as the scraper is rotated for scraping of the entire circumferential extent of the T-zone.

Ayre, himself, recognized that a more reliable diagnosis could be obtained if the scraping sample was not the only sample obtained from a patient. He proposed that at least two separate sampling operations be performed — one using his scraper and another using a separate instrument for obtaining an additional sample directly from the endocervical canal. In fact, research has shown that relying solely on a sample obtained by use of an Ayre scraper can result in a false negative rate of as high as about 30%.

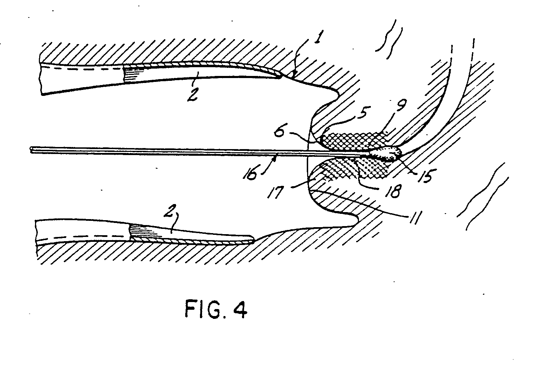

Methods for obtaining samples directly from the endocervical canal are shown in Figures 3 and 4. In the method of Figure 3, the narrow forward end of a pipette 13 is inserted into the endocervical canal. Preferably the tip of the pipette is positioned at about the external os, but it is difficult to position the pipette precisely so that sometimes the tip of the pipette is inserted almost up to the internal os 14 as shown in Figure 3. Suction is applied drawing mucus containing exfoliated cells into the lumen of the pipette. Published research suggests that carefully performed external os aspiration gives more reliable results than any other known single method. A problem, however, is that the endocervical epithelium, unlike squamous epithelium, is friable and prone to bleeding. Not only is bleeding worrisome to the physician and patient, but a sample containing any appreciable amount of blood cannot be evaluated by the cytologist with confidence. Another problem is that the method of Figure 3 more often should be performed by a physician, whereas the methods of Figures 1 and 2 can be performed by skilled paramedic technicians.

In the method of Figure 4 the soft tip 15 of a saline-moistened cotton-tipped applicator 16 is inserted into the endocervical canal and rotated and moved in and out. While less traumatic than the method of Figure 3, there still is a substantial chance of endocervical mucosal injury and bleeding. Also, cells valuable for diagnostic purposes adhere in the interstices of the cotton fiber. Further, it is difficult to transfer the sample to a slide, and vigorously rubbing the cotton-tipped applicator on the slide distorts the cells making them difficult to evaluate.

Adherence of and damage to cells also is a problem with the methods of Figures 1 and 2 because most scrapers presently used are manufactured from thin strips of wood and cells become trapped in the pores and cracks in the wood. There also is a possibility of abrading the cervix with the irregular edge of a wood scraper.

Two or more of the above conventional methods can be performed in sequence on each patient. Prior research has demonstrated that the combination of prior art methods resulting in the lowest false negative rate, a rate as low as 2%, is the combination of the methods of Figures 2 and 3, that is, the combination of T-zone scraping and external os aspiration. In spite of experts' recommendations that this combination of methods be used, physicians continue to use suboptimal methods, possibly because of the difficulty of performing an external os aspiration. For example, in a recent survey, only 3.1% of pathologists representing 675 cytology laboratories stated that a combined external os aspiration and T-zone scraping sample was a "type of routine gynecological smear" received by their laboratories.

A major problem in obtaining Pap smears is that any cells that are allowed to dry before being fixed become distorted and impossible to evaluate. Under ideal circumstances a sample is fixed almost immediately, within seconds after being obtained. This problem is magnified when a combination of prior art methods is used because usually all of the separate samples are obtained and placed on a slide before any sample is fixed, with the result that at least some air drying occurs.

Slides received at a laboratory show significant variation, illustrating a wide range of sampling techniques and methods of transferring collected cell-containing material to a slide. Individual slides may have a single large blop, multiple streaks, globs or a small spot of material such that it is difficult to examine individual cells, making examination time consuming and sometimes inaccurate.

In addition, a substantial proportion of slides received by a laboratory must be classified as "unsatisfactory" because no diagnosis can be given due to an inadequate amount of cell-containing material and/or significant air drying artifact. The proportion of "unsatisfactory" slides varies widely; for example, in one research study involving ten clinics using the same method, T-zone scraping, only one-half of 1% of the slides from one clinic had to be labeled unsatisfactory, whereas 25.6% of the slides from another clinic had to be labeled unsatisfactory because of an inadequate quantity of well defined cells. In this case, the patient must be scheduled for an additional smear sample to be taken which, in effect, doubles the inconvenience to the patient, the work of the doctor or paramedic and the consequent expense. Also, the patient may be distressed from having been asked to return for an additional smear, regardless of the reassurances that she receives from the physician that an abnormality is unlikely.

Different instruments and methods have been proposed to solve one or more of the problems discussed above. For example, to reduce expense it has been proposed that a sample be obtained by the patient herself, such as by use of a specially designed tampon or a "vaginal irrigation" kit, and mailed directly to the medical laboratory. High false negative rates have been demonstrated for self-obtained samples and, accordingly, use of these methods has been discouraged.

One study, in an attempt to explain the high false negative rate when screening for cervical intraepithelial neoplasia, verified that high numbers of cells are trapped in cotton-tipped applicators and wooden scrapers, whereas relatively few cells are trapped in plastic instruments; yet use of plastic instruments has been discouraged because such instruments have been manufactured with sharp edges that can lacerate the cervix.

Kohl in his U.S. patent Re. 27,915 disclosed a scraping instrument having a narrow aspiration pipette projecting forward from the scraping edge of the instrument for insertion into the endocervical canal. As the narrow projecting end of the pipette is inserted into the canal, the scraper and pipette pivot about a flexible shock-absorbing joint to align the axis of the pipette with the length of the endocervical canal. Possible disadvantages of the Kohl construction include:

the Kohl instrument is intended to be gripped at an annular flange projecting from the proximate end portion of the instrument, which may make the Kohl instrument difficult to manipulate;

as with other known aspiration pipettes, it may be difficult to position the pipette precisely and, during insertion, the sharp narrow forward end may lacerate or puncture the friable endocervical epithelium, causing bleeding;

once the instrument is inserted, substantial dexterity would be required to hold the forward end portion of the instrument substantially stationary while the plunger is retracted to draw mucus into the lumen of the instrument; if the forward end portion of the instrument is not maintained stationary, in-and-out movement of the sharp tip of the pipette may cause bleeding;

the small plunger may not be able to generate sufficient suction to draw viscous cell-containing mucus into the lumen of the tube;

diagnostically valuable cells may be trapped in the corrugations of the flexible joint;

the concave scraping edge of the Kohl instrument is designed for engagement with the vaginal ring of the cervix rather than with the transformation zone; if the T-zone is located even slightly inward of the external os, few, if any, cells from the transformation zone would be collected on the smooth cylindrical periphery of the aspiration pipette; and

scraper lobes project oppositely from the pipette so that as the instrument is rotated cells gather on both lateral sides of the scraper portion of the instrument.

Shute in his United States patent No. 3,088,454 disclosed another type of cervical sampling instrument having a scraper and an aspirator tube. Unlike applicant's instrument, the Shute device is designed to obtain the aspiration sample from the internal os, and as the Shute device is rotated, substantially the entire lengthwise extent of the endocervial canal is scraped or sliced by the serrated edge of a sharp blade which may cause bleeding. The sharp blade is substantially thinner than the cylindrical end of the aspiration tube, forming a pocket or depression in which a sample may be trapped.

Robinson in his United States patent No. 4,043,322 disclosed an aspiration instrument having a forward projecting, sharp-edged blade for obtaining tissue samples from the walls of a uterus.

Crane et al. in their United States patent No. 4,078,656 disclosed a return mailer type package for an Ayre scraper, a cotton-tipped applicator, a slide and an ampule of fixative.

Disclosure of the Invention

The present invention provides a sampling instrument for collecting cells exfoliated from the uterine cervix of a woman and having a generally longitudinally elongated aspirator tube with respective proximate and distal ends, the tube having a through bore enabling mucus in the endocervical canal to be sucked into such bore, the distal end portion of· the tube forming a scraper and having a distal top portion insertable into the aperture of the cervix, a lateral lobe projecting transversely of the length of the tube for engagement against the vaginal ring of the cervix, the tube bore opening substantially at the extremity of said distal tip portion, a concave transition section disposed between and joining the inner portion of the lateral lobe and the proximate portion of said distal tip portion, and a blunt scraping edge forming the distal edge of the lateral lobe and the outer edge of said transition section, the lateral lobe having at least one substantially planar lateral side offset from the tube bore and forming with said scraping edge an angular junction, characterized in that the distal tip portion is a flattened frontal lobe including a blunt-nosed, convexly rounded distal tip, the frontal lobe forming part of a flattened portion of the distal end portion of the tube, which flattened portion also forms the lateral lobe, the frontal lobe projecting forward from the lateral lobe a distance sufficient to position said bore opening at approximately the external os when the lateral lobe is in engagement with the vaginal ring of the cervix, the scraping edge also forming a lateral edge of the frontal lobe, and in that the scraping edge is engageable with the inner margin of the vaginal ring of the cervix and positioned to extend across the area of the transformation zone when the lateral lobe is engaged against the vaginal ring- of the cervix, so as to enable collection of a scraping sample of exfoliated cells from the transformation zone while the distal tip portion of the tube is inserted for collection of an aspiration sample.

Additional, optional features are set out in the dependent claims.

The concave transition section of the scraper between the two lobes can be shaped substantially complementally to the inner margin of the vaginal ring of the cervix.

The blunt scraping edge can extend all the way forward up to a broad and blunt apertured tip of the tube. The lateral lobe of the scraper can decrease in thickness outward from the aspirator tube bore so that the sampler can be molded of plastic material without any substantial "dishes" being formed in its opposite lateral sides. A second scraping edge can be provided at the top of the instrument for obtaining a sample for hormonal evaluation, such as from the lateral vaginal wall. Since such wall is less delicate than the endocervix, the junction between the second scraping edge and the planar lateral side of the scraper can be sharper than the junction adjacent to the first scraping edge.

In use, suction is applied to the proximate end portion of the tube for drawing cell-containing mucus into the tube, followed by rotation of the tube for scraping the entire circumferential extent of the T-zone. The aspiration sample is pooled onto a slide and the scraping sample deposited in the pool for transferring the scraping sample onto the slide. The combined sample is spread thinly and evenly as a monolayer of cells on the slide surface and suitable fixative is applied immediately.

Brief Description of Drawings of the Preferred Embodiment

The details of the preferred embodiment will be described in connection with the accompanying drawings, in which,

Figure 5 is a top perspective of a Pap smear T-zone sampler in accordance with the present invention,

Figure 6 is a top perspective of a kit including the sampler of Figure 5, a cotton-tipped applicator, a glass slide, a protective slide case and a package for those components, as well as alternative suction-generating devices for use with the sampler,

Figure 7 is a fragmentary side elevation of the distal end portion of the sampler of Figure 5,

Figure 8 is an end elevation of the sampler of Figure 5,

Figures 9 and 10 are corresponding, somewhat diagrammatic, generally axial sections of a vaginal cavity illustrating a sampler in accordance with the present invention being used for collecting a sample of cell-containing material, with parts broken away, and Figures 11, 12, 13 and 14 are corresponding, fragmentary, somewhat diagrammatic, top perspectives illustrating the sample being deposited on a glass slide and fixed, and

Figure 15 is a somewhat diagrammatic, generally axial section through a vaginal cavity illustrating an alternative manner of collecting a sample by use of the Pap smear T-zone sampler of Figure 5, with parts broken away.

## Definition

As used herein, "lobe" means a blunt nosed projection that is at least somewhat flattened in that it has at least one substantially flat side face.

## Best Mode for Carrying Out the Invention

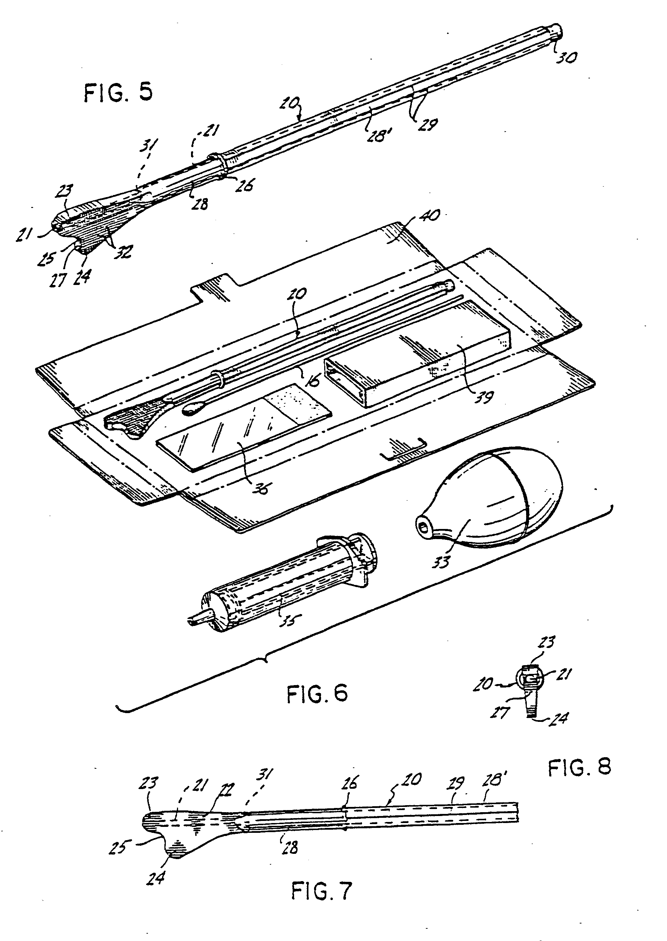

As shown in Figure 5, the Pap smear T-zone sampler of the present invention is a horizontally elongated aspirator tube 20 having a substantially linear axial through bore 21. The distal end portion 22 of the tube is flattened forming a spatulate scraper. In profile, such scraper is shaped substantially the same as an Ayre scraper in having a blunt nosed, forward projecting or frontal lobe 23 and an adjacent downward projecting or lateral lobe 24. A concave transition section 25 of the scraping edge of the scraper is faired into the adjacent edges of the two lobes.

At least one and preferably both of the opposite upright lateral sides of the scraper are planar. A blunt scraping edge 27 extends between such opposite lateral sides and forms the leading edge of the lateral lobe 24, the outer edge of the concave transition section 25 and a lateral or bottom edge of the frontal lobe 23 extending forward. The leading portion of the scraping edge extends forward and then is curved upward to the opening of the bore. The corners forming junctions between the opposite lateral sides of the scraper and its flat scraping edge are angular but, nevertheless, slightly rounded. The planar lateral sides of the scraper include planar marginal portions adjacent to the forward projecting portion of the scraping edge.

A circumferential rib 26 divides the long barrel of the tube into a cylindrical stem 28 carrying the spatulate scraper and a long straight handle portion 28'. Such handle portion is of hexagonal cross section, forming longitudinally extending grip-promoting ridges 29. The relatively short proximate end portion 30 of the tube is cylindrical.

The bore 21 of the tube opens at about the center of the blunt convexly rounded tip of the scraper frontal lobe 23. Throughout the length of the scraper portion 22, such bore is of uniform, but small, diameter. Proceeding toward the proximate end of the tube, the diameter of the bore increases abruptly forming an annular step 31. Throughout the length of the barrel of the tube, the diameter of the bore is uniform, but large in comparison to the diameter of the distal portion of the bore extending through the spatulate scraper.

It is preferred that the aspirator tube be injection molded from plastic material. Also it is preferred that both sides of the flattened spatulate scraper be planar. However, injection molded plastic material has a tendency to shrink in the center of a large thick area forming a characteristic depression or "dish". By tapering the spatulate

scraper from top to bottom, that is, by gradually and uniformly decreasing the thickness of the scraper outward toward the tip of the lateral lobe, as shown in Figure 8, shrinking of the plastic material at the center of the lateral lobe is less of a problem and both sides of the scraper will be substantially planar rather than having substantial central depressions.

Preferably the planar side faces of the scraper are slightly hydrophilic, which can be achieved by adding a hydrophilic substance to the plastic material from which the scraper is formed or, as shown in the drawings, by texturing such sides by molding short criss-crossed ribs 32 integrally in the sides of the scraper as best seen in Figure 5.

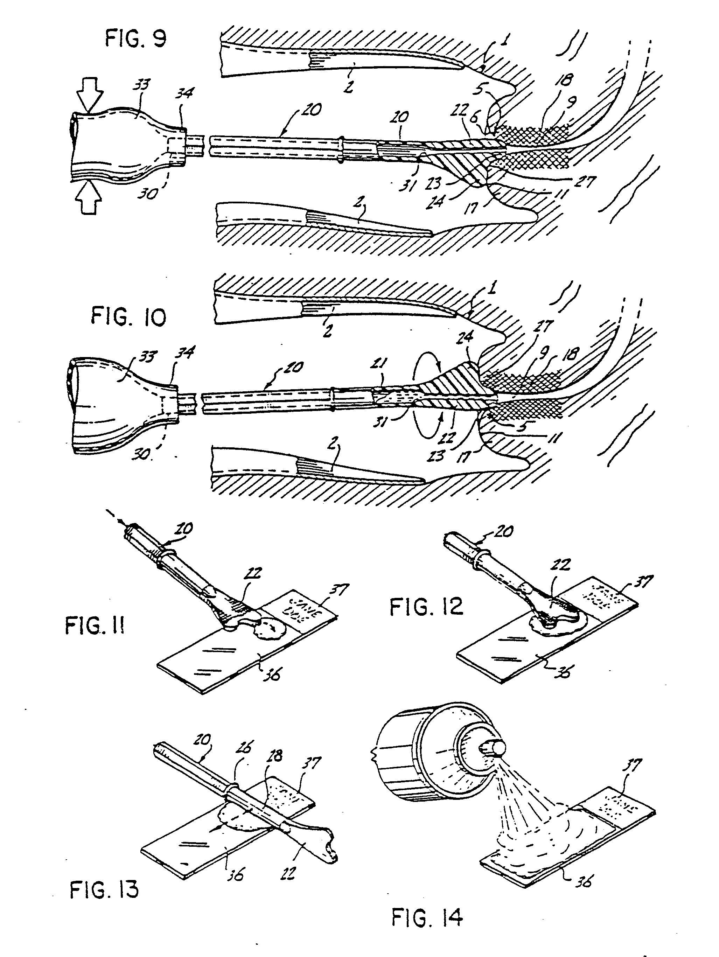

As shown in Figures 9 and 10, in a simple "one-step" operation involving only a single insertion of the Pap smear T-zone sampler of the present invention into the vagina, both an external os endocervical aspiration sample and a T-zone scraping sample can be obtained. The sampler is inserted lengthwise into the vagina 1 distended by a conventional speculum 2 and the broad rounded tip of the frontal lobe 23 is substantially self-centering in the endocervical canal 9 without fear of lacerating or puncturing the cervix. The leading edge of the scraper lateral lobe 24 is located for engagement with the vaginal ring 11 of the cervix to position the apertured tip of the frontal lobe slightly inward of the external os 6. The scraping edge 27 of the scraper is in engagement with the inner margin of the vaginal ring 11 of the cervix and extends inward past the T-zone 5.

Suction is applied to the tube at its proximate end portion, such as by use of a rubber squeeze bulb 33 having its apertured tip 34 snugly fitted over the cylindrical proximate end portion 29 of the tube in sealing engagement. Alternatively, the flared, distal, lumen-forming end portion of a conventional syringe, such as the syringe 35 shown in Figure 6, can be fitted inside the bore of the cylindrical proximate end portion of the tube so that the syringe can be used as a suction-generating device. In either case, the suction is concentrated in the endocervical canal because the distal end portion of the tube bore is of small diameter. Even viscous cell-containing mucus will be drawn into the tube.

While the aspirator tube itself is disposable after use, preferably the suction-generating device is reusable and, accordingly, it is preferred that no mucus be drawn into the suction-generating device, which could contaminate future samples. Since, in the barrel of the tube, the tube bore is of large diameter, a substantial amount of mucus can be drawn into the tube without entering the suction-generating device. In addition, the abrupt step 31 of the bore causes a turbulent flow tending to retain the mucus in the distal end portion of the tube bore, as opposed to a laminar flow which could result in mucus flowing horizontally into the suction-generating device.

After the aspiration sample has been drawn into the tube, the tube is rotated at least one full turn as the scraping edge 27 of the spatulate scraper is held gently against the inner margin of the cervix. The substantially angular but slightly rounded junction between the scraping edge and the leading lateral side of the scraper facilitates collection of freshly exfoliated cells on such leading lateral side. The entire circumferential extent of the T-zone is scraped for collecting freshly exfoliated cells. The scraping sample adheres to the leading side of the scraper because the sides of the scraper are textured or slightly hydrophilic.

An alternative to the two sequential sample-collecting steps described above is to obtain the aspiration sample as the scraper is being rotated, in which case cell-containing material accumulating at the rotating leading side of the scraper may be drawn into the tube bore with mucus from the endocervical canal. Another alternative is to rotate the tube for obtaining the scraping sample first, followed by withdrawing the tube slightly and then applying suction for obtaining the aspiration sample so that exfoliated cells from the T-zone that did not adhere to the leading side of the scraper will be drawn into the tube bore.

Regardless of the sample-collecting method that is used, in rapid sequence the aspirated sample and the scraped sample are deposited on a glass slide 36 and fixed, as shown in Figures 11 through 14. As indicated in Figure 11, first the aspiration sample is pooled onto the slide immediately adjacent to its frosted end 37. Next the scraped sample is transferred to the slide by gently rubbing the leading side of the scraper in the pool as illustrated in Figure 12. Since such side is planar, it can be laid flat against the slide assuring that substantially all cells will be transferred. The combined sample is spread substantially uniformly and thinly on the upper surface slide, as illustrated in Figure 13, by butting the circumferential rib 26 of the tube against a longitudinal edge of the slide with the cylindrical sample-spreading stem 28 resting flatly on the upper surface of the slide and moving the tube lengthwise of the slide away from its frosted end. Immediately following spreading of the sample, the sample is fixed, such as by use of a conventional spray-type fixative as illustrated in Figure 14.

The entire sample transferring and fixing operation can be performed in a matter of a few seconds to that there will be no appreciable air drying of either sample. Each slide should have a thin monolayer of well preserved cells, making microscopic examination and diagnosis quick and reliable.

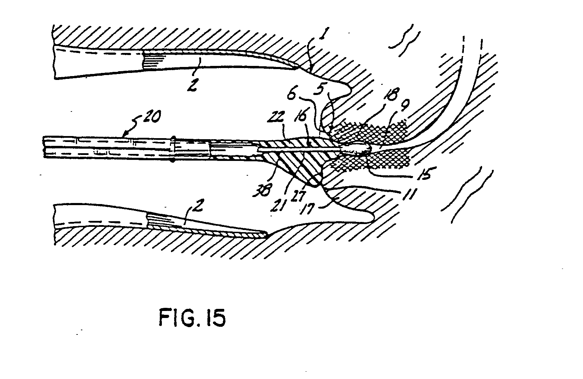

While the combination of external os aspiration and T-zone scraping is the most reliable sampling method for detecting the presence of abnormal cells, in some women there simply is not enough mucus at the external os that an aspiration sample can be obtained. The so-called "dry cervix" is particularly prevalent in peri- or postmenopausal women. Nevertheless, as illustrated in Figure 15, the Pap smear T-zone sampler of the present invention still can be used in a simple one-step operation for obtaining samples of exfoliated cells both from the endocervical canal and the T-zone, even from a woman having "dry cervix".

As shown in Figure 15, the shaft 38 of a cotton-tipped applicator 16 can be inserted into the narrow distal end portion of the bore 21 of the tube 20 up to the soft tip 15 of the applicator. Preferably the diameter of the tube bore is only slightly greater than the diameter of the shaft so that the cotton-tipped applicator is received in the tube bore in snug engagement for firmly connecting the applicator to the aspirator tube.

In use, the aspirator tube still is substantially self-centering as it is inserted lengthwise into the vagina 1 for inserting the tip of the cotton-tipped applicator into the endocervical canal 9. When fully inserted, the scraping edge 27 of the flattened spatulate scraper portion 22 of the tube is in engagement with the T-zone 5. As the tube is rotated for obtaining a T-zone scraping sample, simultaneously exfoliated cells in the endocervical canal are collected on the tip of the cotton-tipped applicator. After at least one full revolution, the tube is withdrawn and, quickly, the cotton-tipped applicator and the leading side of the scraper are rubbed gently against a glass slide for transferring cellular material to the slide. The combined samples are fixed immediately.

The present invention also can be used for obtaining a sample for hormonal evaluation, which sample preferably is obtained from the proximal third of the lateral vaginal wall. For example, following the sample obtaining, transferring and fixing operation described with reference to Figure 9 through 14 or Figure 15, the top edge of the scraper opposite its lateral lobe can be rubbed gently against the lateral vaginal wall between the blades of the speculum. This sample from the lateral vaginal wall can be deposited on a separate slide or at one end portion of the slide containing the cervical scrape sample.

As illustrated in Figure 6, preferably the present invention is provided in a kit including: the Pap smear T-zone sampler 20 of the present invention, which usually will be used to obtain an external os aspiration sample and T-zone scraping sample as shown in Figures 9 and 10; a long shafted cotton-tipped applicator 16, which can be used for wiping away excess mucus from the vaginal portion of the cervix or which can have its shaft shortened for insertion into the bore of the sampler as illustrated in Figure 15; a slide 36 for receiving a sample taken by use of the sampler of the present invention; a rigid slide case 39 for protecting the slide in transit to a medical laboratory; and a folding cardboard package 40 which may be of the return mailer type. Also required for use of the present invention is a suction-generating device; either a rubber squeeze bulb 33 or a plastic syringe 35 can be provided separately. Similarly, spray fixative is preferred and also can be provided separately.

With respect to the preferred dimensions for the invention, to assure adequate suction by use of a rubber squeeze bulb or a syringe; the diameter of the narrow distal end portion of the tube bore should be no greater than about 1/8 inch (.32 cm). As discussed above, preferably the diameter of the narrow portion of the bore is substantially the same as the diameter of the shaft of a cotton-tipped applicator which, for applicators currently available, is about 3/32 inch (.24 cm). The diameter of the larger proximate end portion of the bore should be at least about 1-1/2 times, preferably about 2 times, the diameter of the narrow distal end portion of the bore for creating the abrupt step between the two bore portions and for storing a substantial quantity of mucus.

The transverse thickness of the spatulate scraper should be small, preferably no greater than about twice the diameter of the narrow portion of the tube bore, because there is less chance of a "dish" resulting in a thin section of injection molded plastic material than in the thicker section. Nevertheless, the scraper must be thick enough that the lateral walls of the tube bore will not break or bend appreciably during use of the instrument. In the preferred embodiment, the scraper is tapered uniformly from its thickest top portion, which is about 3/16 inch (.48 cm) thick, to its thinnest bottom portion, which is about 3/32 inch (.24 cm) thick, as shown in Figure 8.

The upright height of the frontal lobe is large in comparison to the diameter of the narrow portion of the tube bore to eliminate the possibility of perforating the cervix. Preferably the height of such lobe, that is, the upright dimension as shown in Figure 7, is at least twice the diameter of the narrow portion of the tube bore, in the preferred embodiment about 1/4 inch (.64 cm) which is between 2 and 3 times the preferred bore diameter. In addition, the tip of the frontal lobe should be blunt and rounded, having a radius of curvature at least equal to about the diameter of the narrow portion of the tube bore. In the preferred embodiment, the tip of the frontal lobe is substantially semicircular, the radius of curvature being about 1/8 inch (.32 cm).

The leading edge of the lateral lobe must project outward a distance sufficient for engagement with the vaginal ring of the cervix and in the preferred embodiment such lobe projects outward about 7/16 inch (1.1 cm) from the axis of the tube bore. In addition, such leading edge must be located rearward from the apertured tip of the frontal lobe a distance such that the apertured tip of the frontal lobe is in close proximity to the external os when the leading edge of the lateral lobe is in engagement with the vaginal ring of the cervix. In the preferred embodiment, the leading edge of the lateral lobe is located about 1/4 inch (.64 cm) rearward of the tip of the frontal lobe.

The substantially angular corners forming junctions between the scraper lateral side faces and the T-zone scraping edge, which includes the adjacent edges of the frontal and lateral lobes and the concave transition section between the lobes, must be rounded sufficiently as to prevent abrading the cervix yet should not be so rounded as to prevent a good scraping effect for collecting exfoliated cells. Preferably the radius of curvature of each of such corners is about .01 inch (.25 mm), and should be no greater than about .03 inch (.76 mm). The top edge of the scraper, which may be used to obtain a sample from the lateral vaginal wall, can have somewhat sharper longitudinally extending corners because the vaginal wall is less friable and prone to bleeding than the cervix. Preferably the radius of curvature of each upper longitudinally extending corner is about .001 inch (0.25 mm) or less.

With respect to the cylindrical proximate end portion of the tube, the outside diameter and the length of such end portion must be sufficient for firm sealing engagement in the aperture of a conventional rubber squeeze bulb. In the preferred embodiment, the outside diameter of the proximate end portion is about 1/4 inch (.64 cm) and such end portion is about 11/32 inch (.87 cm) long. Similarly, the inside diameter of the proximate end portion must be such as to fit over the distal, lumen-forming tip of a conventional syringe in snug sealing engagement. A diameter of about 5/32 or 3/16 inch (.40 or .48 cm) for the distal end portion of the tube bore meets this requirement.

Finally the length and diameter of the long straight handle portion of the tube should be sufficient that the tube can be manipulated easily. In the preferred embodiment, the barrel of the tube is about 6 inches (15 cm) long and the distance between opposite flat sides of the handle portion is slightly greater than 1/4 inch (.64 cm) such that the handle portion is about the same size and is the same shape as a pencil, assuring easy handling of the instrument.

The sampler can be injection molded from clear polypropylene plastics material, in which case the sampler is sufficiently inexpensive that it can be thrown away after use.

**Claims**

1. A sampling instrument for collecting cells exfoliated from the uterine cervix of a woman and having a generally longitudinally elongated aspirator tube (20) with respective proximate and distal ends (30 and 22), the tube (20) having a through bore (21) enabling mucus in the endocervical canal to be sucked into such a bore, the distal end portion (22) of the tube (20) forming a scraper and having a distal tip portion insertable into the aperture of the cervix, a lateral lobe (24) projecting transversely of the length of the tube (20) for engagement against the vaginal ring of the cervix, the tube bore (21) opening substantially at the extremity of said distal tip portion, a concave transition section (25) disposed between and joining the inner portion of the lateral lobe (24) and the proximate portion of said distal tip portion, and a blunt scraping edge (27) forming the distal edge of the lateral lobe (24) and the outer edge of said transition section (25), the lateral lobe having at least one substantially planar lateral side offset from the tube bore (21) and forming with said scraping edge (27) an angular junction, characterized in that the distal tip portion is a flattened frontal lobe (23) including a blunt-nosed, convexly rounded distal tip, the frontal lobe forming part of a flattened portion of the distal end portion (22) of the tube (20), which flattened portion also forms the lateral lobe (24), the frontal lobe (23) projecting forward from the lateral lobe (24) a distance sufficient to position said bore opening (21) at approximately the external os (6) when the lateral lobe (24) is in engagement with the vaginal ring of the cervix, the scraping edge also forming a lateral edge of the frontal lobe (23), and in that the scraping edge (27) is engageable with the inner margin of the vaginal ring of the cervix and positioned to extend across the area of the transformation zone (5) when the lateral lobe (24) is engaged against the vaginal ring of the cervix, so as to enable collection of a scraping sample of exfoliated cells from the transformation zone while the distal tip portion of the tube (21) is inserted for collection of an aspiration sample.

2. An instrument as claimed in Claim 1, in which the radius of curvature of the periphery of the distal tip of the frontal lobe (23) is at least as great as the diameter of the bore opening (21) such that the width of the frontal lobe (23) is at least twice the diameter of the bore opening (21).

3. An instrument as claimed in Claim 1 or Claim 2, in which the blunt-nosed, convexly rounded distal tip of the frontal lobe (23) is substantially semicircular in profile and the tube bore (21) opens substantially centrally of the blunt-nosed distal tip of the frontal lobe (23).

4. An instrument as claimed in any of Claims 1 to 3, in which the minimum thickness of the scraper (22) in a direction laterally of the tube bore (21) is at least as great as the diameter of the bore opening.

5. An instrument as claimed in any of Claims 1 to 4, in which the scraper (22) has a second longitudinally extending scraping edge forming a laterally opposite edge portion of the scraper (22) for scraping a sample from a lateral wall of the vagina.

6. An instrument as claimed in Claim 5, in which the substantially planar lateral side and the second scraping edge form a junction which is sharper than the junction between such lateral side and the primary scraping edge (27).

7. An instrument as claimed in any of Claims 1 to 6, in which the primary scraping edge (27) includes a forward projecting portion extending distally generally longitudinally of the tube bore (21) and forming the said lateral edge of the frontal lobe (23), and the substantially planar upright side includes a marginal portion adjacent to said forward projecting portion of the primary scraping edge (27) and forming therewith a substantially angular junction facilitating scraping of exfoliated cells.

8. An instrument as claimed in any of Claims 1 to 7 wherein the or each substantially planar upright lateral side extends along the entire length of said scraping edge (27).

9. An instrument as claimed in any of Claims 1 to 8, in which the tube (20) is substantially rigid molded plastic material and the scraper (22) is tapered laterally in the direction of projection of the lateral lobe (24).

10. An instrument as claimed in any of Claims 1 to 9, in which the scraper (22) decreases in thickness from the tube bore (21) toward the tip of the lateral lobe (24).

11. An instrument as claimed in any of Claims 1 to 10, in which the junction between the primary scraping edge (27) of the scraper (22) and the substantially planar lateral side is slightly rounded.

12. An instrument as claimed in Claim 11, in which the radius of curvature of the junction between the primary scraping edge (27) of the scraper (22) and the substantially planar lateral side of the scraper (22) is between .01 inch (approx .25 mm) and .03 inch (approx .76 mm).

**Patentansprüche**

1. Probenentnahmeinstrument zum Sammeln von Zellen, die vom Cervixkanal einer Frau abgeschilfert sind, das ein sich allgemeinen in Längsrichtung erstreckendes Ansaugrohr (20) mit einem hinteren und vorderen Ende (30 und 22) umfaßt, wobei das Rohr (20) eine durchgehende Bohrung (21) aufweist, die es ermöglicht, daß Schleimhaut aud dem Inneren des Cervixkanals in die Bohrung eingesaugt wird, wobei der vordere Endteil (22) des Rohrs (20) einen Schaber bildet mit einem vorderen Spitzen-Teil, daß in die Öffnung der Cervix eingeführt werden kann, einem seitlichen Flügel (24), der sich in Querrichtung, bezogen auf die Länge des Rohrs (20) erstreckt und sich an den Vaginal-Ring der Cervix anlegen kann, wobei sich die Bohrung (21) des Rohrs im wesentlichen am äußersten Ende des Spitzen-Teils öffnet, mit einem konkaven Übergangsbereich (25), der zwischen dem inneren Teil des seitlichen Flügels (24) und dem benachbarten Bereich des vorderen Spitzen-Teils angeordnet ist, und mit einer abgerundeten Schabekante (27), die den vorderen Rand des seitlichen Flügels (24) und den äußeren Rand des Übergangsbereichs (25) bildet, wobei der seitliche Flügel mindestens einfach seitlich in der Ebene gegen die Bohrung (21) des Rohrs versetzt ist und mit der Schabekante (27) eine Winkel-Verbindung bildet, dadurch gekennzeichnet, daß der vordere Spitzen-Teil ein abgeflachter Vorder-Flügel (23) ist, umfassend eine konvex abgerundete stumpfe vordere Spitze, der Vorder-Flügel Teil eines abgeflachten Endes des vorderen Endteils (22) des Rohrs (20) ist, wobei dieses abgeflachte Teil auch den seitlichen Flügel (24) bildet, der Vorder-Flügel (23) von dem seitlichen Flügel (24) ausreichend weit vorsteht, daß die Öffnung der Bohrung (21) sich etwa am äußeren Muttermund (6) befindet, wenn der seitliche Flügel (24) an dem Vaginalring der Cervix anliegt und die Schabekante auch einen seitlichen Rand des Vorder-Flügels (23) bildet, und daß die Schabekante (27) in den inneren Rand des Vaginalringes der Cervix eingreifen kann und so angeordnet ist, daß sie sich über den Bereich der Transformationszone (5) erstreckt, wenn der seitliche Flügel (24) an dem Vaginalring der Cervix anliegt, um es zu ermöglichen, eine Schabeprobe von abgeschilferten Zellen aus der Transformationszone zu entnehmen, während der vordere End-Teil des Rohrs (21) eingeführt wird, um eine angesaugte Probe zu entnahmen.

2. Instrument nach Anspruch 1, bei dem der Krümmungsradius des Umfangs der vorderen Spitze des Vorder-Flügels (23) zumindest eben so groß ist wie der Durchmesser der Öffnung der Bohrung (21), so daß die Breite des Vorder-Flügels (23) zumindest 2 mal so groß ist wie der Durchmesser der Öffnung der Bohrung (21).

3. Instrument nach Anspruch 1 oder 2, bei dem die stumpfe konvex abgerundete vordere Spitze des Vorder-Flügels (23) ein im wesentlichen halbkreisförmiges Profil besitzt und die Bohrung (21) des Rohrs sich im wesentlichen in der Mitte der stumpfen vorderen Spitze des Vorder-Flügels (23) öffnet.

4. Instrument nach einem der Ansprüche 1 bis 3, bei dem die minimale Dicke des Schabers (22) in Richtung seitlich von der Bohrung (21) des Rohres zumindest eben so groß ist wie der Durchmesser der Öffnung der Bohrung.

5. Instrument nach einen der Ansprüche 1 bis 4, bei dem der Schaber (22) eine zweite längs verlaufende Schabekante besitzt, die ein seitlich gegenüberliegendes Kantenteil des Schabers (22) bildet zur Entnahme einer Schabeprobe von der Seitenwand der Vagina.

6. Instrument nach Anspruch 5, bei dem die im wesentlichen flache Seite und die zweite Schabekante eine Verbindung bilden, die achärfer ist als die Verbindung zwischen dem Seitenteil und der ersten Schabekante (27).

7. Instrument nach einem der Ansprüche 1 bis 6, bei dem die erste Schabekante (27) einen nach vorn ragenden Teil umfaßt, der sich nach vorne allgemein längs der Bohrung (21) des Rohrs erstreckt und die erwähnte Seitenkante des Vorder-Flügels (23) bildet, und die im wesentlichen flache nach oben stehende Seite einen Randteil umfaßt, angrenzend an den nach vorn ragenden Teil der ersten Schabekante (27), und damit im wesentliche eine Winkel-Verbindung bildet, die ein Abschaben der abgeschliferten Zellen erleichtert.

8. Instrument nach einem der Ansprüche 1 bis 7, bei dem die oder jede im wesentlichen planare, seitlich abstehende Seite sich über die gesamte Länge der Schabekante (27) erstreckt.

9. Instrument nach einem der Ansprüche 1 bis 8, bei dem das Rohr (20) im wesentlichen starr aus einem Kunststoffmaterial gebildet ist und der Schaber (22) seitlich in der Richtung des seitlichen Flügels (24) konisch zuläuft.

10. Instrument nach einem der Ansprüche 1 bis 9, bei dem die Dicke des Schabers (22) von der Bohrung (21) des Rohrs nach dem Ende des seitlichen Flügels (24) hin abnimmt.

11. Instrument nach einem der Ansprüche 1 bis 10, bei dem die Verbindung zwischen der ersten Schabekante (27) des Schabers (22) und der im wesentlichen flachen seitlichen Seite hin leicht gerundet ist.

12. Instrument nach Anspruch 11, bei dem der Krümmungsradius der Verbindung zwischen der ersten Schabekante (27) des Schabers (22) und der im wesentlichen flachen seitlichen Seite des Schabers (22) zwischen 0,01 inch (etwa 0,25 mm) und 0,03 inch (etwa 76 mm) beträgt.

**Revendications**

1. Instrument d'échantillonnage pour recueillir les cellules exfoliées du col de l'utérus d'une femme et ayant un tube aspirateur généralement longitudinalement allongé (20) avec des extrémités respectives proximales et distales (30 et 22), le tube (20) étant traversé d'un alésage (21) permettant aux mucosités dans le canal endocervical d'être aspirées dans cet alésage, l'extrémité distale (22) du tube (20) formant un racloir et ayant un bout distal pouvant être inséré dans l'ouverture du col, un lobe latéral (24) faisant saillie transversalement à la longueur du tube (20) pour un engagement contre l'anneau vaginal du col, l'alésage (21) du tube débouchant sensiblement à l'extrémité dudit bout distal, une section concave de transition (25) disposée entre et joignant la partie interne du lobe latéral (24) et la partie proximale dudit bout distal et un bord émoussé de raclage (27) formant le bord distal du lobe latéral (24) et le bord externe de ladite section de transition (25), le lobe latéral ayant au moins un côté latéral sensiblement plan décalé de l'alésage (21) du tube et formant, avec ledit bord de raclage (27), une jonction angulaire, caractérisé en ce que le bout distal est un lobe frontal aplati (23) ayant un bout distal arrondi de manière convex et un nez émoussé, le lobe frontal faisant partie d'une portion aplatie de l'extrémité distale (22) du tube (20), laquelle portion aplatie forme également le lobe latéral (24), le lobe frontal (23) faisant saillie vers l'avant du lobe latéral (24) sur une distance suffisante pour placer ladite ouverture (21) de l'alésage à peu près à l'endroit de l'orifice externe (6) lorsque le lobe latéral (24) est en engagement avec l'anneau vaginal du col, le bord de raclage formant également un bord latéral du lobe frontal (23) et en ce que le bord de raclage (27) peut venir en engagement avec le bord interne de l'anneau vaginal du col et est placé pour s'étendre à travers la surface de la zone de transformation (5) lorsque le lobe latéral (24) est en engagement contre l'anneau vaginal du col, afin de permettre de recueillir un échantillon de raclage de cellules exfoliées de la zone de transformation tandis que le bout distal du tube (21) est inséré pour recueillir un échantillon par aspiration.

2. Instrument selon la revendication 1 où le rayon de courbure du pourtour du bout distal du lobe frontal (23) est au moins aussi important que le diamètre de l'ouverture (21) de l'alésage de manière que la largeur du lobe frontal (23) soit au moins le double du diamètre de l'ouverture de l'alésage (21).

3. Instrument selon la revendication 1 ou la revendication 2 où le bout distal arrondi de manière convexe à nez émoussé du lobe frontal (23) a un profil sensiblement circulaire et l'alésage (21) du tube débouche sensiblement au centre du bout distal à nez émousse du lobe frontal (23).

4. Instrument selon l'une quelconque des revendications 1 à 3 où l'épaisseur minimale du racloir (22) dans une direction latéralement à l'alésage (21) du tube est au moins aussi importante que le diamètre de l'ouverture de l'alésage.

5. Instrument selon l'une quelconque des revendications 1 à 4 où le racloir (22) a un second bord de raclage s'étendant longitudinalement qui forme un bord latéralement opposé du racloir (22) pour racler un échantillon d'une paroi latérale du vagin.

6. Instrument selon la revendication 5 où le côté latéral sensiblement plan et le second bord de raclage forment une jonction qui est plus aiguë que la jonction entre ce bord latéral et le bord principal de raclage (27).

7. Instrument selon l'une quelconque des revendications 1 à 6 où le bord principal de raclage (27) comprend une partie faisant saillie vers l'avant s'étendant distalement généralement longitudinalement à l'alésage (21) du tube et formant ledit bord latéral du lobe frontal (23), et le côté dressé sensiblement plan comprend une partie marginale adjacente à ladite partie faisant saillie vers l'avant du bord principal de raclage (27) et formant avec elle une jonction sensiblement angulaire facilitant le raclage des cellules exfoliées.

8. Instrument selon l'une quelconque des revendications 1 à 7 où le ou chaque côté latéral dressé sensiblement plan s'étend sur toute la longueur dudit bord de raclage (27).

9. Instrument selon l'une quelconque des revendications 1 à 8 où le tube (20) est en matière plastique moulée sensiblement rigide et le racleur (22) est latéralement effilé dans la direction de la protubérance du lobe latéral (24).

10. Instrument selon l'une quelconque des revendications 1 à 9 où le racloir (22) diminue en épaisseur de l'alésage (21) du tube vers le bout du lobe latéral (24).

11. Instrument selon l'une quelconque des revendications 1 à 10 où la jonction entre le bord principal de raclage (27) du racloir (22) et le côtéral sensiblement plan est légèrement arrondie.

12. Instrument selon la revendication 11 où le rayon de courbure de la jonction entre le bord principal de raclage (27) du racloir (22) et le côté latéral sensiblement plan du racloir (22) est compris entre 0,01 pouce (environ 0,25 mm) et 0,03 pouce (environ 0,76 mm).

FIG. 1

FIG. 2

FIG. 3

FIG. 4

0 050 632

FIG. 5

FIG. 6

FIG. 8

FIG. 7

3

FIG. 9

FIG. 10

FIG. 11

FIG. 12

FIG. 13

FIG. 14

FIG. 15